(19) **Europäisches Patentamt**
**European Patent Office**
**Office européen des brevets**

(11) **EP 3 068 365 B1**

(12) **EUROPEAN PATENT SPECIFICATION**

(45) Date of publication and mention
of the grant of the patent:
**27.06.2018  Bulletin 2018/26**

(51) Int Cl.:
*A61K 8/25* *(2006.01)*  *A61K 8/34* *(2006.01)*
*A61Q 11/00* *(2006.01)*  *A61K 8/9789* *(2017.01)*
*A61K 8/02* *(2006.01)*

(21) Application number: **14777329.5**

(22) Date of filing: **30.09.2014**

(86) International application number:
**PCT/EP2014/070876**

(87) International publication number:
**WO 2015/071023 (21.05.2015 Gazette 2015/20)**

(54) **ORAL CARE COMPOSITIONS**

MUNDPFLEGEMITTEL

COMPOSITIONS DE PROTECTION ORALE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **14.11.2013  EP 13192805**

(43) Date of publication of application:
**21.09.2016  Bulletin 2016/38**

(73) Proprietors:
• **Unilever PLC, a company registered in England and
Wales under company no. 41424 of
London EC4Y 0DY (GB)**
Designated Contracting States:
**CY GB IE MT**
• **Unilever N.V.**
**3013 AL Rotterdam (NL)**
Designated Contracting States:
**AL AT BE BG CH CZ DE DK EE ES FI FR GR HR
HU IS IT LI LT LU LV MC MK NL NO PL PT RO RS
SE SI SK SM TR**

(72) Inventors:
• **ASHCROFT, Alexander Thomas**
**Wirral**
**Merseyside CH63 3JW (GB)**
• **BRENNAN, Lee James**
**Wirral**
**Merseyside CH63 3JW (GB)**
• **WILSON, William John**
**Wirral**
**Merseyside CH63 3JW (GB)**

(74) Representative: **Tansley, Sally Elizabeth**
**Unilever PLC**
**Unilever Patent Group**
**Colworth House**
**Sharnbrook**
**Bedford**
**Bedfordshire MK44 1LQ (GB)**

(56) References cited:
**WO-A1-2013/033090      WO-A1-2013/160023
US-A1- 2009 269 376**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### Field of the Invention

The present invention is concerned with oral care compositions. More particularly, the present invention is concerned with oral care compositions containing activated citrus fibre and particulate silica abrasive.

### Background of the Invention

Abrasives for use in oral care compositions such as dentifrices, are required to be effective in removing extrinsic stains, dental plaque and food debris which builds up on the pellicle on the surface of teeth.

In general, the efficiency of physical removal of stain, plaque and food debris can be increased by using an abrasive having increased abrasivity, or by increasing the level of abrasive incorporated into the composition. However, both these approaches also increase the risk that tooth surfaces may be damaged.

Accordingly, there is a continuing need for oral care compositions that demonstrate satisfactory levels of cleaning, yet are not unduly abrasive and damaging to the teeth.

The present inventors have found that the cleaning efficiency of an oral care composition comprising particulate silica abrasive may be surprisingly enhanced by the inclusion of activated citrus fibre.

Citrus fibres and their uses for structuring of foodstuffs and personal care compositions are described in US2004/0086626 and US2009/269376. The compatibility of an activated citrus fibre structured liquid detergent composition with cleaning and care enzymes is described in WO2012/052306. Its use with cationic deposition polymer for anti-dandruff shampoo is disclosed in WO2012/019934.

US 7,981,855 discloses detergent liquid surfactant compositions comprising up to 15 wt% surfactant, including at least 1% anionic surfactant, up to 2 wt% bacterial cellulose (preferably microfibrous cellulose) and from 0.001 to 5 wt% citrus fibres.

### Summary of the Invention

The present invention provides an oral care dentifrice composition suitable for cleaning the surfaces of the oral cavity, according to claim 1.

### Detailed Description of the Invention

Particulate Silica Abrasive

The composition of the invention comprises from 1 to 70% by weight (based on the total weight of the composition) of particulate silica abrasive which is dispersed in the aqueous continuous phase.

Dental silicas can be produced by either gel or precipitation processes, with precipitated silicas being generally less abrasive.

Preferred particulate silica abrasives for use in the invention may be characterised by the specific shape and size of their constituent primary particles.

By "primary particles" is meant individual particles, defined as the smallest discrete particles that can be seen by electron microscopy analysis (such as, for example, individual crystals).

The primary particles may associate under certain conditions to form larger secondary structures such as aggregates or agglomerates. In the context of the present invention the particle size referred to relates to the overall particle size and where the primary particles are agglomerated relates to the particle size of the agglomerates.

Particle (e.g. crystal) size may be determined by standard techniques known to those skilled in the art. A suitable way for determining particle size of the silica is by use of a Malvern Mastersizer[(R)] model S, with a 300 RF lens and MS17 sample presentation unit. This instrument, made by Malvern Instruments, Malvern, Worcestershire uses the principle of Fraunhofer diffraction, utilising a low power He/Ne laser. Before measurement the sample is dispersed ultrasonically at 25 W ultrasound power in demineralised water for 5 minutes to form an aqueous suspension. The Malvern Mastersizer[(R)] measures the volume particle size distribution of the silica. The volume average particle size ($d_{50}$) or 50 percentile is easily obtained from the data generated by the instrument.

Good cleaning performance has been obtained with particulate silicas with a volume average particle size ($d_{50}$ as described above) from 1 to 15 microns. more preferably ranging from 1.5 to 10 microns, most preferably from 2.5 to 8 microns. Commercially available examples include those materials sold by the company PQ Corporation under the trade names AC33, AC43, AC77. These are precipitated silica abrasives having a volume average particle size ($d_{50}$ as described above) of about 5, 4 and 9 microns respectively.

Citrus fibre

The aqueous continuous phase of the composition of the invention is structured with a matrix of activated citrus fibre particles.

Citrus fibre particles are obtainable from the albedo of citrus fruits. The albedo is generally defined as the white, inner part of the peel, consisting of spongy tissue, containing a matrix of thin walled cells with intercellular air spaces and a few vascular bundles. According to a typical preparation procedure, citrus fibre particles are prepared by taking the insoluble plant cell wall material from lemons and limes, after the juice and oil have been extracted, and then milling, drying and sieving this material. The citrus fibre particles may be further purified with extra washing steps before drying, milling and sieving. Particle size generally ranges from 10 to 500 micron. The particles do not dissolve in water due to their high content of cellulose and hemicellulose but they show excellent swelling properties.

Citrus fibre is termed 'fibre' because of its dietary fibre composition (i.e. the cellulose, hemicellulose and pectin from cell walls) rather than the morphology of its particles. Microscopy studies on citrus fibre particles typically show a heterogeneous mixture of particles having various sizes and shapes. Whilst some of the particles are fibrous in nature, many are spherical. Generally, the majority of the material consists of aggregated lumps of cell walls and cell wall debris. However, a number of tube-like structures with an open diameter of about 10 microns, often arranged in clusters, can be identified. These are water transport channels that are mainly located in the peel of citrus fruits, and termed xylem vessels. The xylem vessels consist of stacks of dead cells, joined together to form relatively long tubes, 200 to 300 microns long. The outsides of the tubes are reinforced by lignin, which is often laid down in rings or helices, preventing the tubes from collapse due to the capillary forces acting on the tube walls during water transport.

A preferred source of citrus fibre particles for use in the composition of the invention is Herbacel AQ Plus citrus fibre. This material is commercially available as a dried powder from Herbafood Ingredients GmbH, Germany. It has a total (soluble and insoluble) fibre content of greater than 80% and soluble fibre content of greater than 20%. It is supplied as a fine dried powder and has a water binding capacity of about 20 kg water per kg of powder.

The term "activation" in the context of the present invention generally means a process in which the citrus fibre particles are hydrated and dispersed. This process swells and breaks up the particles and enables them to form a structuring matrix when incorporated into aqueous media (such as the aqueous continuous phase of the composition of the invention).

In a typical activation process, dried powdered citrus fibre is activated by high shear dispersion at a low concentration in water to form a premix. It is advantageous to include a preservative into the premix.

The shear should not be high enough to lead to defibrillation. If a high-pressure homogeniser is used it should be operated between 200 and 600 bar. The more shear that is applied the less dense the resulting particles. Whilst the morphology is changed by the high shear, the aggregate size appears not to be changed. The fibres break down and then fill the water phase. The shear also rubs loose the outer parts of the cell walls and these are able to form a matrix that structures the water outside of the volume of the original fibre.

An activated citrus fibre premix may alternatively be made by milling using a high shear mixer, such as a Silverson. The premix may be passed through several sequential high-shear stages in order to ensure full hydration and dispersal of the citrus fibre.

The premix may be left to hydrate further (age) after the high shear dispersal. The activated premix is preferably used fresh.

The level of activated citrus fibre in a premix preferably lies in the range 1 to 5%, more preferably 1.5 to 2.5% by total weight citrus fibre (on a dry basis) based on the total weight of the premix. The concentration of citrus fibre in the pre-mix depends on the ability of the equipment to deal with the higher viscosity due to higher concentrations. Preferably the amount of water in the premix is at least 20 times greater than the amount of citrus fibres, more preferably at least 25 times even as much as 50 times. It is advantageous that there is excess water in order to hydrate the citrus fibre fully.

Preferred premixes have a measured yield stress of at least 70 Pa measured using an Anton Paar serrated cup and bob geometry at 25°C.

The amount of citrus fibre in the final oral care composition according to the invention ranges from 0.05 to 5%, preferably from 0.1 to 4%, more preferably from 0.2 to 3% by total weight citrus fibre (on a dry basis) based on the total weight of the composition.

Product Form

A product form in the context of the present invention is a dentifrice. The term "dentifrice" generally denotes formulations which are used to clean the surfaces of the oral cavity. The dentifrice is an oral composition that is not intentionally swallowed for purposes of systemic administration of therapeutic agents, but is applied to the oral cavity, used to treat the oral cavity and then expectorated. Typically the dentifrice is used in conjunction with a cleaning implement such as a toothbrush, usually by applying it to the bristles of the toothbrush and then brushing the accessible surfaces of the oral cavity. In general, a dentifrice may take various physical forms such as solid, semi-solid or liquid (or a combination thereof). For the purposes of this invention the dentifrice is preferably in the form of a paste or a gel (or a combination thereof).

A dentifrice composition according to the invention will contain, as the aqueous continuous phase, a mixture of water and polyhydric alcohol in various relative amounts, with the amount of water ranging from 10 to 45% by weight (based on the total weight of the dentifrice) and the amount of polyhydric alcohol ranging from 30 to 70% by weight (based on the total weight of the dentifrice).

Typical polyhydric alcohols for use in a dentifrice composition according to the invention include humectants such as glycerol, sorbitol, polyethylene glycol, polypropylene glycol, propylene glycol, xylitol (and other edible polyhydric alcohols), hydrogenated partially hydrolyzed polysaccharides and mixtures thereof.

A dentifrice composition according to the invention will generally contain further ingredients to enhance performance and/or consumer acceptability.

For example, the dentifrice may comprise additional abrasive materials. Other abrasive materials will generally be present in an amount of from 0.5 to 75% by weight based on the total weight of the dentifrice. Suitable other abrasive materials include, calcium carbonate, dicalcium phosphate, tricalcium phosphate, calcined alumina, sodium and potassium metaphosphate, sodium and potassium pyrophosphates, sodium trimetaphosphate, sodium hexametaphosphate, particulate hydroxyapatite and mixtures thereof.

Furthermore, the dentifrice may also contain additional binders or thickening agents (in addition to the activated citrus fibre particles described above). Such other binders or thickening agents will generally be present in an amount of in an amount of from 0.5 to 10% by weight based on the total weight of the dentifrice. Suitable binders or thickening agents include carboxyvinyl polymers (such as polyacrylic acids cross-linked with polyallyl sucrose or polyallyl pentaerythritol), hydroxyethyl cellulose, hydroxypropyl cellulose, water soluble salts of cellulose ethers (such as sodium carboxymethyl cellulose and sodium carboxymethyl hydroxyethyl cellulose), natural gums (such as carrageenan, gum karaya, guar gum, xanthan gum, gum arabic, and gum tragacanth), finely divided silicas, hectorites, colloidal magnesium aluminium silicates and mixtures thereof.

Furthermore, the dentifrice will usually contain a surfactant in an amount of from 0.2 to 5% by weight based on the total weight of the dentifrice. Suitable surfactants include anionic surfactants, such as the sodium, magnesium, ammonium or ethanolamine salts of $C_8$ to $C_{18}$ alkyl sulphates (for example sodium lauryl sulphate), $C_8$ to $C_{18}$ alkyl sulphosuccinates (for example dioctyl sodium sulphosuccinate), $C_8$ to $C_{18}$ alkyl sulphoacetates (such as sodium lauryl sulphoacetate), $C_8$ to $C_{18}$ alkyl sarcosinates (such as sodium lauryl sarcosinate), $C_8$ to $C_{18}$ alkyl phosphates (which can optionally comprise up to 10 ethylene oxide and/or propylene oxide units) and sulphated monoglycerides. Other suitable surfactants include nonionic surfactants, such as optionally polyethoxylated fatty acid sorbitan esters, ethoxylated fatty acids, esters of polyethylene glycol, ethoxylates of fatty acid monoglycerides and diglycerides, and ethylene oxide/propylene oxide block polymers. Other suitable surfactants include amphoteric surfactants, such as betaines or sulphobetaines. Mixtures of any of the above described materials may also be used.

Compositions of the present invention may also contain further optional ingredients customary in the art such as fluoride ion sources, anticalculus agents, buffers, flavouring agents, sweetening agents, colouring agents, opacifying agents, preservatives, antisensitivity agents and antimicrobial agents.

To clean the surfaces of the oral cavity, the composition according to the invention is topically applied to the oral cavity surfaces to be cleaned.

Preferably the composition according to the invention is topically applied to the oral cavity surfaces to be cleaned and then agitated by brushing and/or rinsing.

Therefore the invention also provides a composition according to claim 5 for use in method of cleaning the surfaces of the oral cavity comprising the following steps:

a) topically applying the composition according to the invention to the oral cavity surfaces to be cleaned;

b) agitating the composition over the oral cavity surfaces by brushing.

The invention is further illustrated with reference to the following, non-limiting Examples.

### Examples

An activated citrus fibre premix was prepared, having ingredients as shown in Table 1 below.

**Table 1**

| Material | % as supplied | Weight (g) |
|---|---|---|
| Demineralised water | 97.92 | 1958.4 |
| Proxel GXL | 0.08 | 1.6 |
| Herbacel AQ Plus citrus fibre[1] | 2.00 | 40.0 |
| [1] Powdered dried citrus fibre (ex Herbafood Ingredients GmbH, Germany) | | |

The demineralised water was stirred using an agitator stirrer with overhead drive operated at 160 rpm. The Proxel GXL preservative was added. Then Herbacel AQ Plus citrus fibre was added gradually to ensure no clumping. Stirring was continued for a further 15 minutes to allow the fibres to swell sufficiently prior to the activation stage. The activation stage was carried out by high pressure homogenisation (HPH) at 500 barg.

Three dentifrice formulations were prepared using the activated citrus fibre premix described above. Sufficient freshly made premix was added to a mixer to give the required level of activated citrus fibre in the finished composition and it was milled for 10 minutes. The remaining ingredients of the dentifrice formulations were then added. A homogenous dispersion was made using an in-line Silverson (L5T), operating at 9000 rpm with a residence time of 2 minutes.

The ingredients of the resulting dentifrice formulations are shown in Table 2 below. The level of citrus fibre in the formulation is quoted on a dry basis. Numbered Examples represent formulations according to the invention. Lettered Examples are comparative examples (not according to the invention).

**Table 2**

| Ingredient | Ex. A | Ex. 1 | Ex. 2 | Ex. 3 | Ex. B | Ex. C | Ex. D |
|---|---|---|---|---|---|---|---|
| Water | to 100 | to 100 | to 100 | to 100 | to 100 | to 100 | to 100 |
| Sorbitol | 40 | 40 | 40 | 40 | 63 | 63 | 63 |
| Sodium lauryl sulphate | 1.5 | 1.5 | 1.5 | 1.5 | 2 | 2 | 2 |
| Herbacel AQ Plus citrus fibre[1] | 2.5 | 1.5 | 1.5 | 1.5 | | | |
| Abrasive silica AC77 | | 10 | | | 10 | | |
| Abrasive silica AC33 | | | 5 | | | 5 | |
| Abrasive silica AC43 | | | | 5 | | | 5 |
| Thickening silica | | | | | 8 | 8 | 8 |
| Polyethylene Glycol | | | | | 4 | 4 | 4 |
| Flavour | | | | | 1 | 1 | 1 |
| Sodium Carboxymeth ylcellulose | | | | | 0.6 | 0.6 | 0.6 |

Abrasive silica AC77 ex PQ Corporation has a particle size of approximately 9 microns.

Abrasive silica AC33 ex PQ Corporation has a particle size of approximately 5 microns.

Abrasive silica AC43 ex PQ Corporation has a particle size of approximately 4 microns.

The PCR was measured for each of the above formulations. PCR was measured using a method based on that described by Pickles et al.(International Dental Journal 55 (2005), pp. 197-202). This model uses enamel slabs cut from bovine central incisors, embedded in methacrylate resin. The enamel surfaces are smoothed by hand using an alumina paste on a glass block and lightly acid etched in order to facilitate stain accumulation and adherence. The enamel blocks are placed in an incubator set at a constant temperature of 50°C and slowly rotated to alternate between immersion in a staining broth (consisting of tea, coffee and mucin) and air drying. The broth is changed daily and after five days the slabs are removed and washed with distilled water to remove any loose debris. The stained slabs are then brushed in a mechanical brushing machine with distilled water to remove any loosely adhered stain. They are then dried and the L* values ($L^*_{stained}$) from the CIE L*a*b* colour system are measured with a chroma meter in L*a*b* mode. To assess cleaning performance, the stained specimens are then mounted in the mechanical brushing machine and the required load applied to each brush. The test composition is dispersed in an aqueous diluent to form a slurry (typically 38.5g test composition and 61.5g of distilled water) and the stained specimens brushed for a set number of brush strokes with 10ml of slurry. After brushing, the enamel slabs are rinsed with distilled water, dried and the L* values ($L^*_{brushed}$) are re-measured. In the final stage, all traces of stain are removed from the enamel slabs using flour of pumice, on a soft cloth using a grinder/polisher. The enamel slabs are then rinsed with distilled water, dried and the L* values ($L^*_{pumiced}$) are measured and recorded. The percentage of the stain removed by the test composition compared to full removal by pumice (hereinafter referred to as the pellicle cleaning ratio or PCR) may be calculated using the following equation:

$$PCR = [(L^*_{brushed})-(L^*_{stained})/ (L^*_{pumiced})-(L^*_{stained})] \times 100$$

The PCR results after 800 brush strokes are shown in Table 3 below.

**Table 3**

| Test Formulation | Mean 800 (s.d.) |
| --- | --- |
| Example A | 8.22 (3.85) |
| Example 1 | 44.95 (6.26) |
| Example 2 | 52.93 (14.93) |
| Example 3 | 64.31 (6.58) |
| Example B | 36.06 (9.85) |
| Example C | 35.28 (10.13) |
| Example D | 47.94 (9.71) |

It can be seen from these results that the activated citrus fibre (Example A) has little or no cleaning ability on its own. However, when it is combined with silica particles of the claimed particle size (Examples 1 - 3), the cleaning performance is surprisingly enhanced, versus the control examples B-D.

**Claims**

1.  An oral care dentifrice composition suitable for cleaning the surfaces of the oral cavity, the composition comprising:

    an aqueous continuous phase including from 10 to 45% by weight water (based on the total weight of the dentifrice) and from 30 to 70% by weight polyhydric alcohol (based on the total weight of the dentifrice) of one or more polyhydric alcohols;
    from 1 to 70% by weight (based on the total weight of the composition) of particulate silica abrasive in which the particulate silica abrasive has a volume average particle size (dso) ranging from 1 to 15 microns and which is dispersed in the aqueous continuous phase, **characterised in that** the aqueous continuous phase is structured with 0.05 to 5% total weight (on a dry basis) based on the total weight of the composition of a matrix of activated citrus fibre particles,
    in which activated citrus fibres are hydrated and dispersed, thus enabling them to form a structuring matrix when incorporated into aqueous media.

2.  An oral care composition according to claim 1, in which the particulate silica abrasive has a volume average particle size (dso) ranging from 2.5 to 8 microns.

3.  A dentifrice according to claims 1 or 2, which further comprises a surfactant in an amount of from 0.2 to 5% by weight based on the total weight of the dentifrice.

4.  An oral care composition according to any preceding claim, in which the amount of citrus fibre ranges from 0.2 to 3% by total weight citrus fibre (on a dry basis) based on the total weight of the composition.

5.  Composition according to any one of claims 1 to 4 for use in a method of cleaning the surfaces of the oral cavity, the cleaning method comprising the following steps:

    a) topically applying a composition according to any one of claims 1 to 4 to the oral cavity surfaces to be cleaned;

b) agitating the composition over the oral cavity surfaces by brushing.

**Patentansprüche**

1.  Mund- und Zahnpflegezusammensetzung, die zum Reinigen der Oberflächen der Mundhöhle geeignet ist, wobei die Zusammensetzung umfasst:

    eine kontinuierliche wässrige Phase, die von 10 bis 45 Gewichts-% Wasser (bezogen auf das Gesamtgewicht des Zahnpflegemittels) und von 30 bis 70 Gewichts-% mehrwertigen Alkohol (bezogen auf das Gesamtgewicht des Zahnpflegemittels) eines oder mehrerer mehrwertiger Alkohole enthält,
    von 1 bis 70 Gewichts-% (bezogen auf das Gesamtgewicht der Zusammensetzung) teilchenförmiges Siliziumdioxid-Schleifmittel, in welchem das teilchenförmige Siliziumdioxid-Schleifmittel eine volumengemittelte Teil-chengröße ($d_{50}$), die von 1 bis 15 Micron reicht, aufweist und welche in der kontinuierlichen wässrigen Phase dispergiert ist, **dadurch gekennzeichnet, dass** die kontinuierliche wässrige Phase mit insgesamt 0,05 bis 5 Gewichts-% (auf Trockenbasis), bezogen auf das Gesamtgewicht der Zusammensetzung, einer Matrix von aktivierten Citrusfaserteilchen strukturiert ist,
    worin die aktivierten Citrusfasern hydratisiert und dispergiert sind, so dass sie in die Lage versetzt werden, eine strukturierende Matrix zu bilden, wenn sie einem wässriges Medium einverleibt werden.

2.  Mundpflegezusammensetzung nach Anspruch 1, in der das teilchenförmige Siliziumdioxid-Schleifmittel eine volu-mengemittelte Teilchengröße ($d_{50}$) aufweist, die von 2,5 bis 8 Micron reicht.

3.  Zahnpflegemittel nach Anspruch 1 oder 2, das des Weiteren ein Tensid in einer Menge von 0,2 bis 5 Gewichts-%, bezogen auf das Gesamtgewicht des Zahnpflegemittels, umfasst.

4.  Mundpflegezusammensetzung nach einem vorhergehenden Anspruch, in welchem die Menge der Citrusfasern von 0,2 bis 3%, als Gesamtgewicht der Citrusfaser (auf Trockenbasis), bezogen auf das Gesamtgewicht der Zusammensetzung, reicht.

5.  Zusammensetzung nach irgendeinem der Ansprüche 1 bis 4 zur Verwendung in einem Verfahren des Reinigens der Oberflächen der Mundhöhle, wobei das Reinigungsverfahren die folgenden Schritte umfasst:

    a) örtliches Auftragen einer Zusammensetzung nach irgendeinem der Ansprüche 1 bis 4 auf die zu reinigenden Oberflächen der Mundhöhle,
    b) Bewegen der Zusammensetzung über die Oberflächen der Mundhöhle durch Bürsten.

**Revendications**

1.  Composition de dentifrice pour l'hygiène buccale appropriée pour le nettoyage des surfaces de la cavité buccale, la composition comprenant :

    une phase continue aqueuse comprenant de 10 à 45 % en poids d'eau (en se basant sur le poids total du dentifrice) et de 30 à 70 % en poids d'un polyol (en se basant sur le poids total du dentifrice) d'un ou plusieurs polyols ;
    de 1 à 70 % en poids (en se basant sur le poids total de la composition) de silice particulaire abrasive, la silice particulaire abrasive ayant une taille de particule moyenne en volume ($d_{50}$) comprise dans la plage allant de 1 à 15 microns et qui est dispersée dans la phase continue aqueuse, **caractérisée en ce que** la phase continue aqueuse est structurée avec de 0,05 à 5 % en poids total (sur une base sèche) en se basant sur le poids total de la composition d'une matrice de particules de fibres d'agrumes activées,
    dans laquelle les fibres d'agrumes activées sont hydratées et dispersées, ce qui leur permet ainsi de former une matrice structurante lorsqu'elles sont incorporées dans un milieu aqueux.

2.  Composition pour l'hygiène buccale selon la revendication 1, dans laquelle la silice particulaire abrasive a une taille de particule moyenne en volume ($d_{50}$) comprise dans la plage allant de 2,5 à 8 microns.

3.  Dentifrice selon la revendication 1 ou 2, qui comprend en outre un tensioactif en une quantité de 0,2 à 5 % en poids en se basant sur le poids total du dentifrice.

4. Composition pour l'hygiène buccale selon l'une quelconque des revendications précédentes, dans laquelle la quantité de fibre d'agrumes est comprise dans la plage allant de 0,2 à 3 % en poids total de fibre d'agrumes (sur une base sèche) en se basant sur le poids total de la composition.

5. Composition selon l'une quelconque des revendications 1 à 4, destinée à être utilisée dans une méthode de nettoyage des surfaces de la cavité buccale, la méthode de nettoyage comprenant les étapes suivantes consistant à :

a) appliquer de manière topique une composition selon l'une quelconque des revendications 1 à 4 sur les surfaces de la cavité buccale à nettoyer ;
b) agiter la composition sur les surfaces de la cavité buccale par brossage.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20040086626 A
- US 2009269376 A
- WO 2012052306 A
- WO 2012019934 A
- US 7981855 B

### Non-patent literature cited in the description

- **PICKLES et al.** *International Dental Journal,* 2005, vol. 55, 197-202